# EUROPEAN PATENT SPECIFICATION

(11) **EP 0 819 763 B1**
(45) Date of publication and mention of the grant of the patent: **17.11.2004**
(21) Application number: 96907660.3
(22) Date of filing: 27.03.1996
(51) Int. Cl.: C12N 15/12, C07K 14/435, A61K 38/17

(54) **ENGINEERED ACARID ALLERGEN AND PROCESS FOR PRODUCING THE SAME**
GENTECHNOLOGISCH HERGESTELLTES MILBENANTIGEN UND HERSTELLUNGSVERFAHREN
ALLERGENE D'ACARIEN OBTENU GRACE AU GENIE GENETIQUE ET SON PROCEDE DE FABRICATION

(30) Priority: 28.03.1995 JP 9323695
(43) Date of publication of application: 21.01.1998
(73) Proprietor: ASAHI BREWERIES, LTD., Tokyo 104 (JP); TORII PHARMACEUTICAL CO., LTD., Tokyo 103 (JP); THE NIKKA WHISKY DISTILLING CO., LTD., Tokyo 107 (JP)
(72) Inventor: NISHIYAMA, Chiharu, Asahi Breweries Ltd., Outa-ku, Tokyo 143 (JP); YUUKI, Toshifumi, Asahi Breweries Ltd., Outa-ku, Tokyo 143 (JP); OKUMURA, Yasushi, Asahi Breweries Ltd., Outa-ku, Tokyo 143 (JP)
(74) Representative: Chapman, Paul William
(86) International application number: PCT/JP1996/000791
(87) International publication number: WO 1996/030539

(56) References cited:
- EP-A- 0 445 971
- WO-A-94/05790
- JP-A- 6 016 695
- JP-A- 6 253 851
- JP-A- 7 095 887
- PATENT ABSTRACTS OF JAPAN vol. 018, no. 204 (C-1189), 11 April 1994 (1994-04-11) & JP 06 007186 A (ASAHI BREWERIES LTD;OTHERS: 01), 18 January 1994 (1994-01-18)

## Description

### Technical field

The present invention relates to a modified allergen which is obtained by altering a major allergen (Der f II) of house dust mites by gene engineering, and to a method for production of the modified allergen. The modified allergen obtained by the production method can be utilized as a medicine for treating allergic diseases.

It is considered that many allergic diseases are due to several kinds of symptoms which are developed by sensitization of antigen causing the diseases, in which an IgE antibody specific for allergen in blood serum and tissue is produced, the antibody is exposed again to the antigen, and the antibody reacts with the antigen in each tissue. Particularly, an immediate type reaction is caused by the combination of antigens with IgE antibodies on mast cells and basophiles followed by crosslinking the IgE antibodies, and the subsequent release of several kinds of chemical mediators from the mast cells or the basophiles.

There is a method for controlling the binding between the antigen and the IgE antibody as a method for treating the allergic diseases. If the binding between the antigen and the IgE antibody is controlled, the crosslinking among the IgE antibodies on the mast cells or the basophiles and the release of chemical mediators are controlled to have an effect on the treatment.

On the other hand, it appears that allergic diseases such as bronchial asthma, childhood asthma, atopic dermatitis and the like are mainly caused by allergen from mites living in house dust. Several kinds of proteins of major mite allergens have been identified as major mite allergens (Platts-Mills et al., J. Allergy Clin. Immunol., 80, 755(1987)). Further, a mass production method of a purified major mite allergen has been disclosed (Yuuki et al, Japanese J. Allergology, 39, 557(1990) and Japanese Patent publication No 0̸3254683). In addition, the allergen changed a part of the above purified major mite allergen and a production method thereof has been filed. (Japanese Patent publication No. 0̸6253851).

However, the proteins of the major allergens of mites, which have been reported and identified, show problems of an allergy reaction, namely anaphylactic shock, in the hyposensitization therapy, because the activity of these allergens is high.

On the other hand, if the modified major mite allergen, which has lower IgE-binding activity or lower allergen activity than wild type allergens and inhibits the binding of the antigen (Der f II) and the IgE antibody, is obtained, it is possible to provide an effective medicine for treating allergic diseases. The medicine does not show the anaphylactic shock of allergy reaction caused by antigen administration, and it does not have an effect on the other immunity system since the medicine is specific to the antigen Modified major mite allergens have been disclosed in Japanese Patent publication Nos. 06253851 and 07095887. In the former application, there are problems of maintenance and stability of immunogenicity because molecular structure is much changed by amino acids substituents. In the latter application, it is not enough to minimize the structure change and to lower the allergen activity because a modification position of the allergen is not satisfactorily specified and alanine only is used as an amino acid substituent.

The inventors of the present invention have found that, when an amino acid of a specified part of major mite allergen Der f II which is already disclosed is replaced with other similar amino acid to minimize the structure change, IgE-bonding activity can be changed. It is found that there is no difference between the modified major mite allergens and those of a wild-type as to the activity inhibit the antigen (Der f II) from binding to IgE.

An object of the present invention is to provide a method of mass production of the modified major mite allergen Der f II in which amino acid replacements are introduced by gene engineering. Namely, the present invention aims to produce a material utilizable as a medicine for treating allergic diseases.

### Disclosure of Invention

The present invention is characterized in a method that a major allergen (Der f II) of house dust mites (Dermatophagoides farinae) is modified by gene engineering to replace amino acid residues with other amino acid residues, procaryotes or eucaryotes being transformed with a replication vector containing a gene which codes the modified major mite allergen. Der f II, are cultured and the modified major mite allergen is obtained from the culture.

It has been disclosed that the major mite allergen Der f II has six cysteine residues, and has three intramolecular crosslinkages (between 8th and 119th, between 21st and 27th, and between 73rd and 78th of amino acid residues) by disulfide bonds (Nishiyama et al., International Archives of Allergy and Immunology, 101, 159(1993)). Further, the effect of IgE-binding activities is disclosed by preparing a modified Der f II which is obtained by lacking one of those disulfide bonds in the molecular by gene engineering technique (Japanese Patent Publication No. 0̸6253851). According to the report, the disulfide bond between 8th and 119th is most important for IgE-binding activity.

The present inventors have repeatedly investigated and found that an area around the disulfide bond between 73rd cysteine and 78th cysteine has an effect on the IgE-binding. Moreover, they have found that it is possible to lower the IgE-binding capability significantly by replacing an amino acid with an amino acid having a property very close to it. For example, 7th aspartic acid can be replaced with glutamic acid or asparagine as well as above-mentioned alanine. Though 19th aspartic acid is replaced with glutamic acid, the change of the IgE-binding capability is not observed. However, when the aspartic acid is replaced with asparagine, it is found that the IgE-binding capability is much lowered and the capability is affected by the electric charge at the 19th amino acid residue. In addition, for example, when 9th alanine is replaced with leucine, the binding activity is about 30% of original activity. On the other hand, when the alanine is replaced with proline as shown in the present invention, it is found that the binding activity is lowered to 10% or less. Among the mutants having the binding activity which is much lowered effective mutants are found by an animal experiment of mice developing mite allergy. Thereby, the present invention has been attained by finding modified allergens effective to a hyposensitization treatment for patients of mite allergy.

The modified allergens of the present invention can be produced by any method suitable to the aims of the present invention, preferably by a site-directed mutagenesis method. Although many site-directed mutagenesis methods have been established, a PCR method is handy (Ito et al., Gene, 102, 67(1991). As an example, it is shown in the following that the 7th aspartic acid residue of Der f II protein, is replaced with a glutamic acid residue using the DNA chain as shown in Sequence No. 1-a.

The codon corresponding to the 7th aspartic acid residue is GAT in SEQ ID No: 1. This codon is replaced with a codon corresponding to the glutamic acid, for example, GAG. The oligonucleotide having the same sequence as that of the DNA sequence near the aspartic acid residue, in which only the codon (GAT) of the aspartic acid residue is replaced with a codon (GAG) of the glutamic acid residue. is synthesized (Table 1, F-D7E). This synthesis may be conducted by a well-known method, conveniently by automatic synthesizer (for example, Model 381 DNA synthesizer: manufactured by Applied Biosystems company).

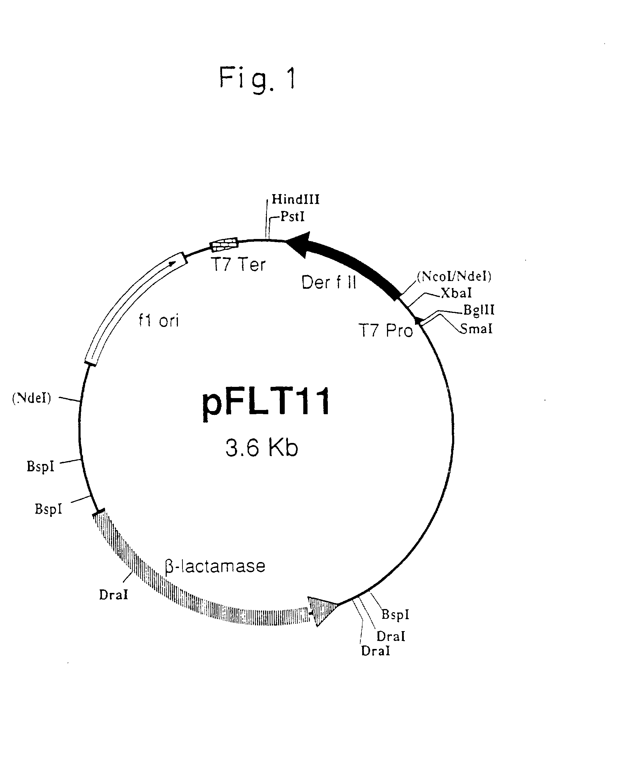

Any DNA fragments including cDNA of the Der f II shown in Sequence No. 1 can be used as the template DNA for amplifying by a PCR method. In this case pFLT 11 (Fig. 1) was used as the template. The synthetic oligonucleotide R 1 (Table I) has the same sequence as that of the region containing a sequence of Hind III recognition site at the downstream of the Der f II-coding region on pFLT 11, PCR was conducted using two synthetic oligonucleotides, above F-D7E and R1 as primers.

After the PCR, the nucleotide sequences of the resulting amplified DNA fragments can be determined by dideoxy method (J. Mol. Biol., 162, 729-773(1982)) and the like.

Thus, modified DNA, which had been certified the introduction of a mutation, is inserted into a cloning site of a suitable expression vector, and a modified Der f II can be expressed. In this expression, any plasmid vector stably existent in E. coli can be used, and conveniently, for example, pGEMEX1 (manufactured by Promega Company) can be used. In this vector, a T7 promoter is used as an expression promoter, and it is known that there is much expression and the recombinant protein accumulated in E. coli as a inclusion body. Many methods can be used in a chain of these operations (Maniatis et al., Molecular Cloning, Cold Spring Harbor Laboratory, 1982).

The DNA can be expressed by using a suitable vector such as YEp13 (Broach et al, Gene, 8, 121-133(1979) in yeast. Using a yeast vector having an expression cassette accompanying a modified Der f II gene which is obtained by the method of the present invention, a suitable yeast cell can be transformed. For the object, the DNA sequence of the present invention must be not controlled by an E. coli promoter, but an eucaryotic promoter, for example, delta P8 and the like (Otake et al., Agric. Biol. Chem., 52, 2753-2762(1988)).

Thus, modified plasmids were prepared by substituting other amino acids except alanine for the 7^{th} amino acid from the N terminal side of the major mite allergen Der f II, respectively, and each plasmid was transformed and expressed in E. coli..

Then, the IgE antibody binding activity of modified Der f II was quantitatively determined. Before the determination, it needs to purify each modified Der f II protein. The outline is as follows. Cells of host E. coli BL 21 carryng above plasmid were harvested after expression. The cells were hypersonically crushed, and the Der f II protein expressed as inclusion body was collected by centrifugation. After dissolving the inclusion body with 6M urea, the solution was dialyzed to a buffer of 20 mM Tris hydrochloride (pH 8.5) to remove urea and refold the protein. Then, the Der f II fraction was purified by anion exchange chromatography. Namely, the fraction including the refolded protein was adsorbed to a DEAE-Toyopearl (manufactured by Tosoh company) column, and eluted with 80 mM NaCl to obtain purified Der f II protein.

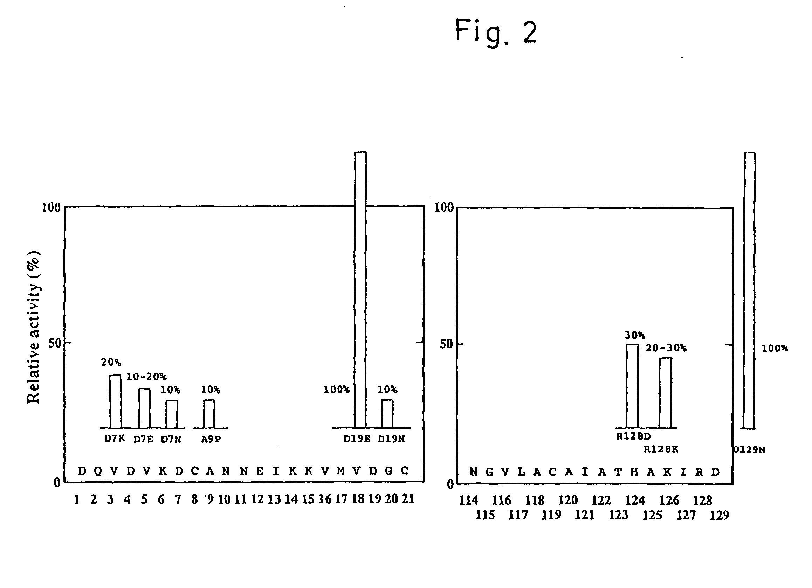

Using thus resulting pure modified Der f II, the IgE-binding activity was quantitatively determined. For this object, a RAST-EIA kit (manufactured by Pharmacia company) is conveniently used. First, a disk of filter paper activated with bromcyanide was immersed in 50 µl of a solution of modified Der f II which was diluted with a buffer solution of 0.1 M boric acid (pH 8.5), and was permitted to stand overnight, and the protein was bound to the filter paper. After washing, the filter paper was immersed in 50 µl of a mite allergic patient's serum which was diluted four times with buffer solution attached to the kit, allowed to leave for two hours at 37°C, and an antigen binding to the filter paper was bound to a human anti Der f II IgE antibodies in the serum. Then, reaction was proceeded in accordance with the reaction protocol of the kit. After all reaction was finished, 420 nm of the absorbance of the sample was an index of the IgE-binding activity. The results are shown in Fig. 2. Modified proteins obtained by substituting alanine, asparagine and glutamic acid for 7 th come to notice. As to 7 th aspartic acid, even if it is replaced by any amino acid, the binding ability with IgE is more lowered in comparison with that of the wild-type Der f II. Accordingly, the necessary condition is that 7 th amino acid is aspartic acid for IgE-binding.

Then, using the modified proteins of Der f II having a lowered IgE-binding activity in an animal experiment, the efficiency was confirmed for the hyposensitization. 10 µg of Der f II and 100 µg of Freund's adjuvant were intraperitoneally administered to male seven-week-old A/J mice to sensisize with rDer f. One mg/ml of modified proteins were intranasally administered to sensitized mice two times a week for three weeks. The same amount of a physiological salt solution was administered to controls. One week after the final administration, the mice were received into a chamber for small animals to inhale five mg/ml of nebulized Der f II for 30 minutes. After 24 hours, the mice were killed by bleeding, followed by bronchial alveolar lavage with 1 ml of PBS. Cytospin preparations were made from 200 µl of bronchial alveolar lavage fluid (abbreviated as BALF hereinafter ), and stained with Dif-quick (Registered Trademark). The samples were observed under a microscope of 400 magnification. and leukocytes (macrophages, neutrophils, eosinophils and lymphocytes) were counted within the range of the microscope to determine the degree of airway inflammation. In non-sensitized mice leukocytes in BALF taken after inhalation of antigen, are little observed except macrophages. On the other hand, in immunized mice, many neutrophils and lymphocytes were observed and the leukocyte count was about 2.5 times higher than that of the nonimmune groups. Namely, in sensitized mice, it was confirmed that the airway inflammation was induced by the inhalation of the antigen. In contrast, in sensitized mice administered either wild-type Der f II or the substituent, leukocyte count was substantially decreased to show the effective hyposensitization. Accordingly, it is proved that modified proteins of Der f II are safe and effective agents for treating allergic diseases, because there is little possibility of an anaphylaxis shock lowering the IgE-binding activity.

### Brief Description of Drawings

Fig. 1 shows pFLT11 which was used for amplification by a PCR method.

Fig. 2 is a graph showing the results that the absorbance of the reaction solution observed at 420 nm in Example 2, and evaluated the value as binding activities of modified proteins of Der f II and human IgE.

### Best Mode for Carrying Out the Invention

The following examples illustrate the present invention in details.

### Example 1 Construction of an expression vector of a Der f II amino acid-substituted mutant

A vector developing a variant that the objective amino acid residue of Der f II had been substituted by the other amino acid was prepared by a site-directed mutagenesis method of PCR. Before conducting the PCR method, oligonucleotides were synthesized as shown in Table 1.

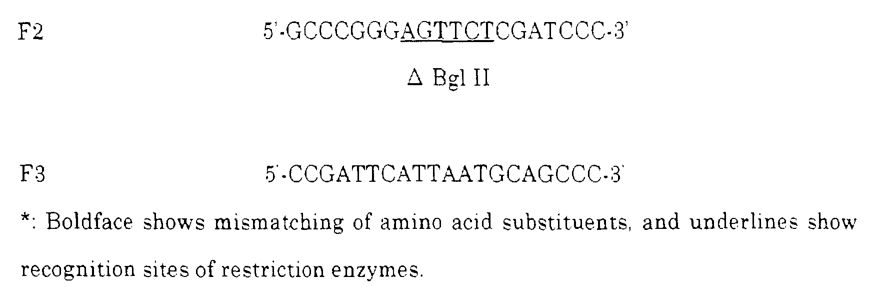

Table 1 Base sequences of synthesized oligonucleotides for preparing mutants
Oligonucleotides | Sequences*
(Mutants) | Nde I
F-D7E (Asp7Glu) | 5'-GCCATATGGATCAAGTCGATGCTAAAGAGTGTGC-3'
F-D7N (Asp7Asn) | 5'-GCCATATGGATCAAGTCGATGCTAAAAATTGTGC-3'
F-D7K (Asp7Lys) | 5'-GCCATATGGATCAAGTCGATGTTAAAAAATGTGC-3'
Hind III
R1 | 5'-ATCAAGCTGGGATTTAGGTG-3'
F1 | 5'-CCCCGCGCGTTGGCCGATTC-3'

When 7th Asp from the N terminal side of Der f II was varied to Glu or Asn, a synthesized oligonucleotide was designed so as to have the same sequence as that of wild-type Der f II except that a codon of the objective amino acid to be varied was changed to a codon of Glu or Asp, and to reintroduce a recognition sequence of restriction enzyme Nde I into an upstream region of the initiation codon. PCR was carried out using expression vector pFLT11 (Japanese patent application No. 5-139793) including cDNA of the wild-type Der f II as a template. Synthesized oligonucleotide R1 having a sequence complementary to a region containing a Hind III recognition sequence of the downstream side of a site which clones Der f II on pFLT11, and the above synthesized oligonucleotide F-D7E or -D7N were used as primers. The PCR solution was prepared by adding each one µg of two primers, F-D7E and R1, or F-D7N and R1, to one ng of the plasmid pFLT11 as a template; adding 10 µl of a 10x reaction solution attached to Taq DNA polymerase (TOYOBO), 10 µl of a 25 mM MgCl₂ solution; adding dATP, dCTP, dGTP and dTTP so that each final concentration is 200 µM in 100 µl of the reaction solution; adding distilled water to adjust the volume of the reaction solution to 100 µl; and adding 2.5 unit of the Taq DNA polymerase. After the resulting solution was left at 94 °C for one minute to change the two-stranded DNA of the template into a single-stranded DNA, and it was left at 37 °C for two minutes to anneal the primer into a one-stranded template, and then the solution was reacted at 72 °C for three minutes to synthesize a complementary DNA by the polymerase. The above steps were repeated 25 cycles to amplify the objective DNA fragments.

After the digestion of the resulting DNA fragments by Nde I and Hind III, the fragments were inserted into the Nde I / Hind III site of plasmid pGEME1-Δ Nde (Japanese Patent Application No. 5-139793) to obtain expression vectors including DNAs of the mutants, pFLT11-D7E and D7N, respectively.

Each 10 ng of these two DNA fragments obtained by the PCR was added to the solution obtained by removing the primer and the polymerase from the previous PCR solution, the mixture was reacted at 94 °C for 10 minutes, cooled gradually to 37 °C for 30 minutes, and maintained at 37 °C for 15 minutes to anneal these two fragments. Subsequently, 2.5 units of Taq polymerase was added, and maintained at 60 °C for three minutes to elongate these two DNA fragments. Then, each 0.5 µg of previous two primers R1 and F1 were added, 20 cycles of PCR were repeated to amplify these fragments. By such operation, two kinds of fragments having the same length were obtained. One fragment had a Bgl II recognition sequence and it was varied at the objective amino acid residue, and the other fragment had the same amino acid sequence as that of the wild type and it was varied so as to be impossible to break at the Bgl II recognition sequence. After these fragments were digested with Bgl II and Hind III, these were linked to pGEMEX1-Δ Nde I which were treated with Bgl II and Hind III, and only vectors were obtained in which the objective mutations were introduced.

### Example 2 Comparison of IgE-binding abilities of the amino acid substituted mutants of Der f II by a RAST-EIA method

After E. coli BL21 transformed by each expression vector of the Der f II mutants constructed in Example 1 (pFLT11-D7E, pFLT11-D7N, and pFLT11-A9P) were grown on an agar medium containing ampicilin (1% bactotryptone, 0.5% yeast extract, 0.5% sodium chloride, 1.5% bactoagar (these units were W/V), 50 µg/ml ampicilin, pH 7.4), the resulting colonies were inoculated to 5 ml of an L liquid medium containing ampicilin (agar was removed from the L agar medium containing ampicilin). After the colonies were cultivated with shaking at 30 °C overnight, the medium was added to 500 ml of an L liquid medium containing ampicilin, and further the solution was cultivated with shaking at 30 °C. When the absorbancy of the solution reached to 0.4, isopropyl-β-thiogalactopyranocide (IPTG) was added to 0.1 mM, the solution was cultivated for more five hours with shaking, and the objective protein was expressed. Then, the E. coli BL21 cells expressing each Der f II mutant were harvested by centrifugation, the cells were broken with a ultrasonic blender, and Der f II mutants expressed as inclusion bodies were collected by centrifugation. The inclusion bodies were refolded by solubilization in a buffer solution containing urea (6M urea. 100 mM Tris hydrochloric acid and 10mM ethylenediaminetetraacetic acid (EDTA), pH 7.5), and by dialysis in a Tris buffer solution (20 mM tris hydrochloric acid, pH 8.5). This solution was provided on an ion exchange column DEAE-Toyopearl (TOSOH) which was equilibrated in the above Tris buffer solution and was eluted by a concentration gradient of sodium chloride (from 0 mM to 100 mM). The fragment eluted at about 80mM of salt concentration was subjected to SDS-PAGE, and a purified sample was obtained at a single band of about 14,000 of molecular weight.

Human IgE-binding activity of the wild-type Der f II and each mutant was determined by using a RAST-EIA kit manufactured by Pharmacia Company. The operation method was as follows. First, a filter paper, which was activated with bromocyan, was dipped in 50 µl of an antigen solution diluted with a buffer solution of 0.1 M boric acid (pH 8.5), and incubated overnight at room temperature to adsorb the antigen. Then, the antigen solution was removed, the filter paper was washed in 500 µl of a solution of 0.1 M sodium hydrogencarbonate and dipped in 250 µl of 1 M β-ethanolamine (pH 9.0), and incubated for three hours at room temperature to prevent nonspecific absorption on the paper by blocking operation. The ethanolamine solution was removed, the paper was washed once in 500 µl of a solution of 0.1 M sodium hydrogencarbonate and three times in 500 µl of a buffer solution of 0.1 M sodium acetate (pH 4.0), and twice in 500 µl of the buffer solution attached to the kit. Adding 50 µl of serum of an allergy patient, which was diluted 4 times with the buffer solution attached to the kit, the paper in the solution was incubated for two hours at 37 °C to combine anti Der f II-IgE in the serum and the antigen adsorbed on the filter paper.

After the antigen-antibody reaction, the serum was removed, the filter paper was washed three times by dipping it for ten minutes in 2.5 ml of the washing solution attached to the kit, 50 µl of an anti-human IgE-rabbit IgG solution labeled with β-D-galactosidase of the kit was added, and the resulting solution was left for 16-20 hours at room temperature to combine the IgE adsorbed to the antigen. After the enzyme-labeled antibody solution was removed, the filter paper was washed as described above, and 200 µl of substrate o-nitrophenol-β-D-galactopyranoside attached to the kit was added to react at 37 °C for two hours. After adding 2 ml of a solution attached to the kit for stopping the enzyme reaction, the absorbance at 420 nm of the reaction solution was determined, the value was evaluated as binding activity of Der f II and human IgE. The results are as shown in Fig. 2. It is recognized that the IgE-binding activity of each Der f II mutants of D7E, and D7N is remarkably lowered in comparison with the wild type Der f II. Namely, if amino acid Asp 7 which was considered to be important for forming a human IgE epitope of Der f II from the experiment of substituting Ala, was replaced with Asn which did not have a negative charge unlike Asp, it was found that the IgE-binding activity was remarkably lowered like the Ala-substituted Der f II. Even if it was replaced with Glu which was an amino acid having a negative charge like Asp, the IgE-binding activity was also lowered.

### Example 3

### A test for evaluating the effectiveness in the hyposensitization treatment of the amino acid substituted mutant of Der f II

### [Materials and Method]

### Immunization

Ten µg of recombinant Der f II (abbreviated as rDer f II hereinafter) and 100 µl of Freund's adjuvant were intraperitoneally administered three times to 24 male A/J mice of seven-week-old each two weeks.

### Test groups

Twenty four immunized mice were classified to a control group of six mice, a rDer f II administration group of six mice, and a D7N administration group of six mice. In addition, as a reaction-negative control group, three mice, which were not immunized, were named as a non- immune group.

### Administration of rDer f II and a mutant substituted by amino acid residues (D7N).

Two weeks after the final immunization, a physiological salt solution of phospholyc acid buffer (abbreviated as PBS hereinafter) to the control group, one mg/ml or rDer f II to the rDer f II administration group, and one mg/ml of D7N to the D7N administration group, were intranasally administered each 20 µl two times per week for three weeks respectively. The nonimmune group was not treated.

### A test for provocating late phase airway inflammation

A week after the final intranasal administration, the mice were received into a chamber for small animals to inhale five mg/ml of nebulized Der f II for 30 minutes. After 24 hours, the mice were killed by bleeding, followed by bronchial alveolar lavage with 1 ml of PBS. Cytospin preparations were made from 200 µl of bronchial alveolar lavage fluid (abbreviated as BALF hereinafter), and stained with Dif-quick (Registered Trademark). The samples were observed under a microscope of 400 magnification, and leukocytes (macrophages, neutrophils, eosinophils and lymphocytes) were counted within the range of the microscope to determine the degree of airway inflammation.

### [Results]

### Numbers of leukocytes in BALF after inhaling rDer f II

In case of the nonimmune group, leukocytes in BALF are little observed except macrophages. On the other hand, in case of the control group of immunized mice, many neutrophil and lymphocytes were observed. In comparison with nonimmune groups, the leukocyte count of the control group was about 2.5 times. Namely, in case of the control group, it was confirmed that the airway inflammation was provoked by the inhale of the antigen. On the contrary, in case of the groups administered rDer f II or the substituent of amino acid residues, D7N, numbers of neutrophils and all leukocyte were decreased in comparison with the control group, so that it was recognized the realization of the hyposensitization to rDer f II by the intranasal administration. The results are shown in Table 2.

**Table 2**

| Number of leukocytes (± standard error) | | | | |
|---|---|---|---|---|
| Test groups | Macrophage | Neutrophil | Eosinophil | Lymphocyte |
| Nonimmune | 43.2±5.4 | 0.3± 0.3 | 0.0 | 0.2±0.2 |
| Control | 47.1±2.4 | 60.0±13.5 | 1.7±1.1 | 2.8±1.3 |
| rDer f II | 42.7±8.7 | 31.5± 8.9 | 3.7±2.2 | 4.1±1.4 |
| D7N | 46.8±7.4 | 35.8±10.5 | 2.9±0.8 | 5.1±3.0 |

### Industrial Applicability

The modified Der f II made by gene technology according to the present invention can be lowered the binding activity of the IgE antibody in the minimum mutation (an amino acid in 129 amino acids) and can be utilized for treating each kind of allergy diseases caused by mites in high safety.

### [Table of Sequences]

SEQ ID No: 1-a
   Sequence length: 390
   Sequence type: nucleic acid
   Strandedness: double
   Topology: linear
   Molecule type: cDNA to mRNA
   Feature
   Original source: *Dermatophagoides farinae*
   Method for determining the feature: E
   Sequence 
1 Asp Gln Val Asp Val Lys Asp Cys Ala Asn Asn Glu Ile Lys Lys Val
1 GAT CAA GTC GAT GTT AAA GAT TGT GCC AAC AAT GAA ATC AAA AAA GTA

17 Met Val Asp Gly Cys His Gly Ser Asp Pro Cys Ile Ile His Arg Gly
49 ATG GTC GAT GGT TGC CAT GGT TCT GAT CCA TGC ATC ATC CAT CGT GGT

33 Lys Pro Phe Thr Leu Glu Ala Leu Phe Asp Ala Asn Gln Asn Thr Lys
97 AAA CCA TTC ACT TTG GAA GCC TTA TTC GAT GCC AAC CAA AAC ACT AAA

49 Thr Ala Lys Ile Glu Ile Lys Ala Ser Leu Asp Gly Leu Glu Ile Asp
145 ACC GCT AAA ATT GAA ATC AAA GCC AGC CTC GAT GGT CTT GAA ATT GAT

65 Val Pro Gly Ile Asp Thr Asn Ala Cys His Phe Val Lys Cys Pro Leu
193 GTT CCC GGT ATC GAT ACC AAT GCT TGC CAT TTT GTC AAA TGT CCA TTG

81 Val Lys Gly Gln Gln Tyr Asp Ile Lys Tyr Thr Trp Asn Val Pro Lys
241 GTT AAA GGT CAA CAA TAT GAT ATC AAA TAT ACA TGG AAT GTG CCG AAA
 
97 Ile Ala Pro Lys Ser Glu Asn Val Val Val Thr Val Lys Leu Ile Gly
289 ATT GCA CCA AAA TCT GAA AAC GTT GTC GTT ACA GTC AAA CTT ATC GGT

113 Asp Asn Gly Val Leu Ala Cys Ala Ile Ala Thr His Gly Lys Ile Arg
337 GAT AAT GGT GTT TTG GCT TGC GCT ATT GCT ACC CAT GGT AAA ATC CGT

129 Asp ***
385 GAT TAA
SEQ ID No: 1-b
   Sequence length: 390
   Sequence type: nucleic acid
   Strandedness: double
   Topology: linear
   Molecule type: cDNA to mRNA
   Feature
   Original source: *Dermatophagoides farinae*
   Method for determining the feature: E
   Sequence 
1 Asp Gln Val Asp Val Lys Asp Cys Ala Asn Asn Glu Ile Lys Lys Val

1 GAT CAA GTC GAT GTT AAA GAT TGT GCC AAC AAT GAA ATC AAA AAA GTA

17 Met Val Asp Gly Cys His Gly Ser Asp Pro Cys Ile Ile His Arg Gly

49 ATG GTC GAT GGT TGC CAT GGT TCT GAT CCA TGC ATC ATC CAT CGT GGT

33 Lys Pro Phe Thr Leu Glu Ala Leu Phe Asp Ala Asn Gln Asn Thr Lys

97 AAA CCA TTC ACT TTG GAA GCC TTA TTC GAT GCC AAC CAA AAC ACT AAA
 
49 Thr Ala Lys Ile Glu Ile Lys Ala Ser Leu Asp Gly Leu Glu Ile Asp
145 ACC GCT AAA ATT GAA ATC AAA GCC AGC CTC GAT GGT CTT GAA ATT GAT

65 Val Pro Gly Ile Asp Thr Asn Ala Cys His Phe Met Lys Cys Pro Leu
193 GTT CCC GGT ATC GAT ACC AAT GCT TGC CAT TTT ATG AAA TGT CCA TTG

81 Val Lys Gly Gln Gln Tyr Asp Ile Lys Tyr Thr Trp Asn Val Pro Lys
241 GTT AAA GGT CAA CAA TAT GAT ATC AAA TAT ACA TGG AAT GTG CCG AAA

97 Ile Ala Pro Lys Ser Glu Asn Val Val Val Thr Val Lys Leu Ile Gly
289 ATT GCA CCA AAA TCT GAA AAC GTT GTC GTT ACA GTC AAA CTT ATC GGT

113 Asp Asn Gly Val Leu Ala Cys Ala Ile Ala Thr His Gly Lys Ile Arg
337 GAT AAT GGT GTT TTG GCT TGC GCT ATT GCT ACC CAT GGT AAA ATC CGT

129 Asp ***
385 GAT TAA
SEQ ID No: 1-c
   Sequence length: 390
   Sequence type nucleic acid
   Strandedness: double
   Topology: linear
   Molecule type: cDNA to mRNA
   Feature
   Original source: *Dermatophagoides farinae*
   Method for determining the feature: E
   Sequence 
1 Asp Gln Val Asp Val Lys Asp Cys Ala Asn Asn Glu Ile Lys Lys Val
1 GAT CAA GTC GAT GTT AAA GAT TGT GCC AAC AAT GAA ATC AAA AAA GTA

17 Met Val Asp Gly Cys His Gly Ser Asp Pro Cys Ile Ile His Arg Gly
49 ATG GTC GAT GGT TGC CAT GGT TCT GAT CCA TGC ATC ATC CAT CGT GGT

33 Lys Pro Phe Thr Leu Glu Ala Leu Phe Asp Ala Asn Gln Asn Thr Lys
97 AAA CCA TTC ACT TTG GAA GCC TTA TTC GAT GCC AAC CAA AAC ACT AAA

49 Thr Ala Lys Ile Glu Ile Lys Ala Ser Leu Asp Gly Leu Glu Ile Asp
145 ACC GCT AAA ATT GAA ATC AAA GCC AGC CTC GAT GGT CTT GAA ATT GAT

65 Val Pro Gly Ile Asp Thr Asn Ala Cys His Phe Met Lys Cys Pro Leu
193 GTT CCC GGT ATC GAT ACC AAT GCT TGC CAT TTT ATG AAA TGT CCA TTG

81 Val Lys Gly Gln Gln Tyr Asp Ala Lys Tyr Thr Trp Asn Val Pro Lys
241 GTT AAA GGT CAA CAA TAT GAT GCC AAA TAT ACA TGG AAT GTG CCG AAA

97 Ile Ala Pro Lys Ser Glu Asn Val Val Val Thr Val Lys Leu Val Gly
289 ATT GCA CCA AAA TCT GAA AAC GTT GTC GTT ACA GTC AAA CTT GTT GGT

113 Asp Asn Gly Val Leu Ala Cys Ala Ile Ala Thr His Ala Lys Ile Arg
337 GAT AAT GGT GTT TTG GCT TGC GCT ATT GCT ACC CAC GCT AAA ATC CGT
 
129 Asp ***

385 GAT TAA
SEQ ID No: 1 (D7E)
   Sequence length: 390
   Sequence type: nucleic acid
   Strandedness: double
   Topology: linear
   Molecule type: cDNA to mRNA
   Feature
   Original source: *Dermatophagoides farinae*
   Method for determining the feature: E
   Sequence 
1 Asp Gln Val Asp Val Lys Glu Cys Ala Asn Asn Glu Ile Lys Lys Val
1 GAT CAA GTC GAT GTT AAA GAG TGT GCC AAC AAT GAA ATC AAA AAA GTA

17 Met Val Asp Gly Cys His Gly Ser Asp Pro Cys Ile Ile His Arg Gly
49 ATG GTC GAT GGT TGC CAC GGT TCT GAT CCA TGC ATC ATC CAT CGT GGT

33 Lys Pro Phe Thr Leu Glu Ala Leu Phe Asp Ala Asn Gln Asn Thr Lys
97 AAA CCA TTC ACT TTG GAA GCC TTA TTC GAT GCC AAC CAA AAC ACT AAA

49 Thr Ala Lys Ile Glu Ile Lys Ala Ser Leu Asp Gly Leu Glu Ile Asp
145 ACC GCT AAA ATT GAA ATC AAA GCC AGC CTC GAT GGT CTT GAA ATT GAT

65 Val Pro Gly Ile Asp Thr Asn Ala Cys His Phe Met Lys Cys Pro Leu
193 GTT CCC GGT ATC GAT ACC AAT GCT TGC CAT TTT ATG AAA TGT CCA TTG
 
81 Val Lys Gly Gln Gln Tyr Asp Ala Lys Tyr Thr Trp Asn Val Pro Lys
241 GTT AAA GGT CAA CAA TAT GAT GCC AAA TAT ACA TGG AAT GTG CCG AAA

97 Ile Ala Pro Lys Ser Glu Asn Val Val Val Thr Val Lys Leu Val Gly
289 ATT GCA CCA AAA TCT GAA AAC GTT GTC GTT ACA GTC AAA CTT GTT GGT

113 Asp Asn Gly Val Leu Ala Cys Ala Ile Ala Thr His Ala Lys Ile Arg
337 GAT AAT GGT GTT TTG GCT TGC GCT ATT GCT ACC CAC GCT AAA ATC CGT

129 Asp ***
385 GAT TAA
SEQ ID No: 2 (D7N)
   Sequence length: 390
   Sequence type: nucleic acid
   Strandedness: double
   Topology: linear
   Molecule type: cDNA to mRNA
   Feature
   Original source: *Dermatophagoides farinae*
   Method for determining the feature: E
   Sequence 
1 Asp Gln Val Asp Val Lys Asn Cys Ala Asn Asn Glu Ile Lys Lys Val
1 GAT CAA GTC GAT GTT AAA AAT TGT GCC AAC AAT GAA ATC AAA AAA GTA

17 Met Val Asp Gly Cys His Gly Ser Asp Pro Cys Ile Ile His Arg Gly
49 ATG GTC GAT GGT TGC CAC GGT TCT GAT CCA TGC ATC ATC CAT CGT GGT

33 Lys Pro Phe Thr Leu Glu Ala Leu Phe Asp Ala Asn Gln Asn Thr Lys
97 AAA CCA TTC ACT TTG GAA GCC TTA TTC GAT GCC AAC CAA AAC ACT AAA

49 Thr Ala Lys Ile Glu Ile Lys Ala Ser Leu Asp Gly Leu Glu Ile Asp
145 ACC GCT AAA ATT GAA ATC AAA GCC AGC CTC GAT GGT CTT GAA ATT GAT

65 Val Pro Gly Ile Asp Thr Asn Ala Cys His Phe Met Lys Cys Pro Leu
193 GTT CCC GGT ATC GAT ACC AAT GCT TGC CAT TTT ATG AAA TGT CCA TTG

81 Val Lys Gly Gln Gln Tyr Asp Ala Lys Tyr Thr Trp Asn Val Pro Lys
241 GTT AAA GGT CAA CAA TAT GAT GCC AAA TAT ACA TGG AAT GTG CCG AAA

97 Ile Ala Pro Lys Ser Glu Asn Val Val Val Thr Val Lys Leu Val Gly
289 ATT GCA CCA AAA TCT GAA AAC GTT GTC GTT ACA GTC AAA CTT GTT GGT

113 Asp Asn Gly Val Leu Ala Cys Ala Ile Ala Thr His Ala Lys Ile Arg
337 GAT AAT GGT GTT TTG GCT TGC GCT ATT GCT ACC CAC GCT AAA ATC CGT

129 Asp ***
385 GAT TAA
SEQ ID No: 3 (D7K)
   Sequence length: 390
   Sequence type: nucleic acid
   Strandedness: double
   Topology: linear
   Molecule type: cDNA to mRNA
   Feature
   Original source: *Dermatophagoides farinae*
   Method for determining the feature: E
   Sequence

1 Asp Gln Val Asp Val Lys Lys Cys Ala Asn Asn Glu Ile Lys Lys Val

1 GAT CAA GTC GAT GTT AAA AAA TGT GCC AAC AAT GAA ATC AAA AAA GTA

17 Met Val Asp Gly Cys His Gly Ser Asp Pro Cys Ile Ile His Arg Gly

49 ATG GTC GAT GGT TGC CAC GGT TCT GAT CCA TGC ATC ATC CAT CGT GGT

33 Lys Pro Phe Thr Leu Glu Ala Leu Phe Asp Ala Asn Gln Asn Thr Lys

97 AAA CCA TTC ACT TTG GAA GCC TTA TTC GAT GCC AAC CAA AAC ACT AAA

49 Thr Ala Lys Ile Glu Ile Lys Ala Ser Leu Asp Gly Leu Glu Ile Asp

145 ACC GCT AAA ATT GAA ATC AAA GCC AGC CTC GAT GGT CTT GAA ATT GAT

65 Val Pro Gly Ile Asp Thr Asn Ala Cys His Phe Met Lys Cys Pro Leu

193 GTT CCC GGT ATC GAT ACC AAT GCT TGC CAT TTT ATG AAA TGT CCA TTG

81 Val Lys Gly Gln Gln Tyr Asp Ala Lys Tyr Thr Trp Asn Val Pro Lys

241 GTT AAA GGT CAA CAA TAT GAT GCC AAA TAT ACA TGG AAT GTG CCG AAA

97 Ile Ala Pro Lys Ser Glu Asn Val Val Val Thr Val Lys Leu Val Gly
289 ATT GCA CCA AAA TCT GAA AAC GTT GTC GTT ACA GTC AAA CTT GTT GGT

113 Asp Asn Gly Val Leu Ala Cys Ala Ile Ala Thr His Ala Lys Ile Arg
337 GAT AAT GGT GTT TTG GCT TGC GCT ATT GCT ACC CAC GCT AAA ATC CGT

129 Asp ***
385 GAT TAA

## Claims

1. A method for producing a modified major mite allergen **characterized in that** it comprises culturing procaryotes or eucaryotes, which have been transformed with a replication vector containing a gene, and collecting the modified major mite allergen from the culture; wherein the gene encodes a modified major mite allergen DerfII, which has been substituted at the 7^{th} amino acid from the N terminal side of the major mite allergen DerfII encoded by the DNA sequence of SEQ ID No: 1a, 1b or 1c, by another amino acid except alanine.

2. A polypeptide comprising or consisting of a modified mite allergen DerfII, which has been substituted at the 7^{th} amino acid from the N-terminal side of the major mite allergen DerfII encoded by the DNA sequence of SEQ ID NO. 1a, 1b or 1c by another amino acid except alanine.

3. A polypeptide comprising an amino acid sequence of SEQ ID No: 1 (D7E), SEQ ID No: 2 (D7N) and SEQ ID No: 3 (D7K).

4. A DNA chain coding any one of polypeptides as claimed in claim 3.

5. A medicine for treating mite allergic diseases comprising the modified mite allergen as claimed in claim 2.

6. A medicine for treating mite allergic diseases comprising the Polypeptide as claimed in claim 3.

7. A medicine for preventing mite allergic diseases comprising the modified mite allergen as claimed in claim 2.

8. A medicine for preventing mite allergic diseases comprising the polypeptide as claimed in claim 3.

## Patentansprüche

1. Verfahren zur Herstellung eines modifizierten Milben-Hauptallergens, **dadurch gekennzeichnet, daß** es die Kultivierung von Prokaryonten oder Eukaryonten, die mit einem ein Gen enthaltenden Replikationsvektor transformiert wurden, und die Gewinnung des modifizierten Milben-Hauptallergens aus der Kultur umfaßt, wobei das Gen für ein modifiziertes Milben-Hauptallergen DerfII codiert, das an der 7. Aminosäure von der N-terminalen Seite des durch die DNA-Sequenz SEQ ID NR. 1a, 1b oder 1c codierten Milben-Hauptallergens DerfII durch eine andere Aminosäure außer Alanin substituiert ist.

2. Polypeptid, das ein modifiziertes Milben-Allergen DerfII, das an der 7. Aminosäure von der N-terminalen Seite des durch die DNA-Sequenz SEQ ID NR. 1a, 1b oder 1c codierten Milben-Hauptallergens DerfII durch eine andere Aminosäure außer Alanin substituiert ist, umfaßt bzw. daraus besteht.

3. Polypeptid, umfassend eine Aminosäure-Sequenz SEQ ID NR. 1 (D7E), SEQ ID NR. 2 (D7N) und SEQ ID NR. 3 (D7K).

4. DNA-Kette, die für eines der Polypeptide nach Anspruch 3 codiert.

5. Medikament zur Behandlung von milbenallergischen Erkrankungen, umfassend das modifizierte Milbenallergen nach Anspruch 2.

6. Medikament zur Behandlung von milbenallergischen Erkrankungen, umfassend das Polypeptid nach Anspruch 3.

7. Medikament zur Verhütung von milbenallergischen Erkrankungen, umfassend das modifizierte Milbenallergen nach Anspruch 2.

8. Medikament zur Verhütung von milbenallergischen Erkrankungen, umfassend das Polypeptid nach Anspruch 3.

## Revendications

1. Procédé de production d'un allergène majeur d'acarien modifié, **caractérisé en ce qu'**il comprend la culture de procaryotes ou d'eucaryotes qui ont été transformés par un vecteur de réplication contenant un gène et la récolte de l'allergène majeur d'acarien modifié dans la culture; dans lequel le gène code pour un allergène majeur d'acarien DerfII modifié, dont le 7^{ème} acide aminé à partir de l'extrémité N-terminale de l'allergène majeur d'acarien DerfII codé par la séquence d'ADN de la SEQ ID N° 1a, 1b ou 1c est remplacé par un autre acide aminé, à l'exception de l'alanine.

2. Polypeptide comprenant ou consistant en un allergène d'acarien DerfII modifié, dont le 7^{ème} acide aminé à partir de l'extrémité N-terminale de l'allergène majeur d'acarien DerfII codé par la séquence d'ADN de la SEQ ID N° 1a, 1b ou 1c est remplacé par un autre acide aminé, à l'exception de l'alanine.

3. Polypeptide comprenant une séquence d'acides aminés de la SEQ ID N° 1 (D7E), la SEQ ID N° 2 ((D7N) et la SEQ -ID N° 3 (D7K).

4. Chaîne d'ADN codant pour l'un quelconque des polypeptides selon la revendication 3.

5. Médicament pour traiter les maladies allergiques dues aux acariens, comprenant l'allergène d'acarien modifié selon la revendication 2.

6. Médicament pour traiter les maladies allergiques dues aux acariens, comprenant le polypeptide selon la revendication 3.

7. Médicament pour prévenir les maladies allergiques dues aux acariens, comprenant l'allergène d'acarien modifié selon la revendication 2.

8. Médicament pour prévenir les maladies allergiques dues aux acariens, comprenant le polypeptide selon la revendication 3.
